# EUROPEAN PATENT SPECIFICATION

(11) **EP 2 148 620 B1**
(45) Date of publication and mention of the grant of the patent: **28.08.2013**
(21) Application number: 08738098.6
(22) Date of filing: 07.05.2008
(51) Int. Cl.: A61B 5/11, G01G 19/44

(54) **WEIGHT AND/OR MOVEMENT SENSING IN A BED**
GEWICHTS- UND/ODER BEWEGUNGSMESSUNG IN EINEM BETT
DETECTION DE POIDS ET/OU DE MOUVEMENT DANS UN LIT

(30) Priority: 14.05.2007 EP 07108179
(43) Date of publication of application: 03.02.2010
(73) Proprietor: Koninklijke Philips Electronics N.V., 5621 BA Eindhoven (NL); Philips Intellectual Property & Standards GmbH, 20099 Hamburg (DE)
(72) Inventor: BRAUERS, Andreas, NL-5656 AE Eindhoven (NL); DORSCHEID, Ralf, NL-5656 AE Eindhoven (NL); JOHNEN, Frank, NL-5656 AE Eindhoven (NL)
(74) Representative: Smit, Frederik Jan
(86) International application number: PCT/IB2008/051790
(87) International publication number: WO 2008/139377

(56) References cited:
- EP-A- 0 322 994
- US-A- 4 793 428
- US-A- 5 780 781

## Description

The invention refers to a sensor arrangement for weight and/or movement sensing in a bed and uses thereof, to a bed and to a method of sensing weight and/or movement of a user in a bed.

The measurement of vital body signs in bed using force or pressure sensors is known. One of the very important parameters that can be measured in bed is the weight of the person. This is especially interesting for patients with a cardiac disease or other patients which are bed ridden. Integrating standard weight sensors into the bed usually means introducing mechanical springs between the subject bearing surface and the ground and measuring their deformation, for example by strain gauges. In the United States patent No. 4 793 428, a hospital bed with an integrated scale is disclosed which is incorporated into the bed between the bed support frame and the patient support section. For reasons of mechanical stability, measuring the weight of a human body, mechanical setups which are at least a couple of centimeter in height, are required. It is a drawback that the height of the scale leads to an elevation of parts of the bed. While in a professional environment (hospital, nursing homes,...) this can be accepted and also compensated to some extent due to the standardized nature of the beds. However, this is a real issue, when an application in the patients' homes is addressed. Here, the sensors should be integratable into the bed of the patient, without compromising the comfort of the patient, and virtually invisible, thus not disturbing the design of the bed. This aim should be achieved irrespective of the specific design of the patient's bed.

A sensor arrangement for weight sensing which can be retrofit to a bed is known from US-A-5,780,781. It comprises a structure attachable between a bedframe and a mattress frame, and weighing sensors, which are placed in the edging areas in which the mattress frame is supported by the bedframe.

It is therefore an objective of the present invention to provide a sensor arrangement for weight and/or movement sensing in a bed, which reduces the drawbacks according to the state of the art.

The above objective is accomplished by a sensor arrangement for weight and/or movement sensing in a bed, comprising a structure attachable between a bedframe and a mattress frame, the structure comprising a first sensor support and a second sensor support, the first sensor support and the second sensor support extending in a space below the mattress frame, where in the space the mattress frame is unsupported by the bedframe, wherein at least one sensor is arranged between the first sensor support and the second sensor support. While this invention is focused on retrofit solutions for standard beds in patients' homes it can also be adapted to the needs of hospital and care beds.

It is an advantage of the sensor arrangement according to the invention, that it can be attached to a bed without significantly enhancing a height of a surface bearing the structure, i.e. the bedframe. Thus the height of the mattress frame above the bedframe is only marginally increased. Nevertheless, the sensor arrangement provides advantageously the mandatory stiffness of a spring, allowing for a quantitative force measurement like weight measurement in the bed. It is a further advantage that no constructive changes to the bed itself are necessary.

The bedframe of the bed is a structural part which has substantially direct and unsprung contact to the ground, i.e. there are essentially no elastic elements between the bedframe and the ground. The bedframe is thus advantageously applicable to accommodate the sensor support. The mattress frame of the bed is, according to the sense of the invention, the frame which is usually supported by the bedframe in any regular bed. The mattress frame carries directly or indirectly the mattress. Preferably, the mattress frame is a slatted frame, but may as well be a frame with a network of springs or even a flat plank. The bedframe usually supports the mattress frame in an edging area or, as the case may be, additionally in an area of a crossbeam. The space below the mattress frame, in which the mattress frame is unsupported by the bedframe, in the sense of the invention, is that space, which is enclosed by a vertical projection of the bedframe or, respectively, the of the supporting area of the bedframe and the mattress frame. This space below the mattress frame is advantageously applicable for measuring the weight and/or vital body signs (VBS). In particular, mechanical setups for measuring the weight of a human body, are advantageously easily accommodated in the unutilized space between the mattress frame and the ground. As a further advantage, the sensors and the sensor supports are located in a hidden position below the bed, the design of which is thus unaffected by the sensor arrangement. The structure, the first sensor support and the second sensor support are any kind of essentially rigid beams which are applicable for carrying the sensor. Preferably, the sensor is a force detecting sensor.

The structure comprises load transmission means for transmitting forces exerted between the mattress frame and the bedframe to the first sensor support and the second sensor support. The structure advantageously only marginally increases the height of the mattress frame above the bedframe. Preferably a dimension of the structure between the bedframe and the mattress frame is substantially smaller than a distance between the first sensor support and the second sensor support. The distance between the first sensor support and the second sensor support is the space which is advantageously utilizable for accommodating the sensor.

According to another preferred embodiment of the invention, the first sensor support is pivotally connected to the second sensor support. The structure comprises in particular a scissor-like mechanism. This embodiment of the structure is particularly advantageous for accommodating one sensor. Preferably, a plurality of sensor arrangements according to this embodiment is attached between the bedframe and the mattress frame.

According to a further preferred embodiment of the invention, the first sensor support and/or the second sensor support is formed as an intermediate frame. The intermediate frames extend across the bed like a longitudinal beam or cross beam between the parts of the bedframe. Preferably, a plurality of sensors is arranged between the first sensor support and the second sensor support.

Another object of the invention is a bed for sensing weight and/or movement of a user, comprising a bedframe and a mattress frame, wherein at least one sensor arrangement according to the invention as described in here before, is attached between the bedframe and the mattress frame. It is an advantage that any suitable standard bed which comprises a bedframe and a preferably detachable mattress frame, is easily equipped with the sensor arrangement. The bed is preferably only temporarily equipped with the sensor arrangement.

In a preferred embodiment of the invention, the bed is retrofit with the sensor arrangement. The integration of the sensor arrangement into the bed may advantageously be done as a retrofit by laymen or service persons with minimum effort and in a very easy procedure.

A further object of the invention is a method of sensing weight and/or movement of a user in a bed, comprising the steps of
transferring forces exerted between a bedframe and a mattress frame of the bed to a space below the mattress frame, where in the space, the mattress frame is unsupported by the bedframe and
measuring the forces by at least one sensor in the space below the mattress frame.

The space below the mattress frame, in which the mattress frame is unsupported by the bedframe, is advantageously applicable for measuring the weight and/or vital body signs.

For the sensing of weight and/or movement, including activity and/or cardiopulmonary performance in a bed, preferably force sensors integrated between the bedframe and the mattress frame are used. The sensors are designed in a way that they can be positioned between the mattress frame and the bedframe without significantly enhancing the height of a mattress bearing surface. Thus, the integration into any bed with bedframe can be easily realized. The measurements do not require any user interaction or even direct contact between user and sensor.

A further object of the invention is a use of a sensor arrangement according to the invention for sensing a weight and/or movements of a user in a bed.

A further object of the invention is a use of a sensor arrangement according to the invention for equipping a bed comprising a bedframe and a mattress frame with a weight and/or movement sensing feature.

These and other characteristics, features and advantages of the present invention will become apparent from the following detailed description, taken in conjunction with the accompanying drawings, which illustrate, by way of example, the principles of the invention. The description is given for the sake of example only, without limiting the scope of the invention.

The reference Figures quoted below refer to the attached drawings.
Fig. 1 illustrates schematically a sensor arrangement and a bed according to the invention.
Figs. 2 and 3 illustrate schematically a first embodiment of the sensor arrangement according to the invention in two views.
Fig. 3 illustrates schematically a second embodiment of the sensor arrangement according to the invention.

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes.

Where an indefinite or definite article is used when referring to a singular noun, e.g. "a", "an", "the", this includes a plural of that noun unless something else is specifically stated.

Furthermore, the terms first, second, third and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the present description and claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

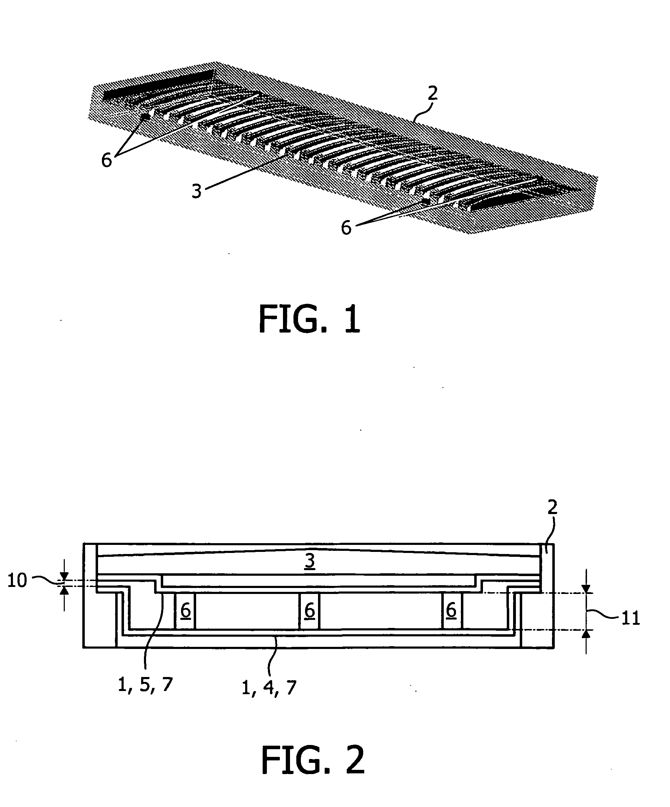

Figure 1 shows a schematic perspective view of a bed according to the invention comprising a sensor arrangement according to the invention. Parts of a bedframe 2 are cut away. Figure 1 illustrates the positions of sensors 6 and the principle of operation of the invention. While a user (not depicted) is lying on the bed, his movements are transferred to a mattress (not shown) and a mattress frame 3 which is a slatted frame 3 in the depicted embodiment. The movements are transferred from the slatted frame 3 to the bedframe 2 which is in contact to the ground, for example by means of legs (not depicted). Even small movements can be detected using appropriately sensitive force sensors 6. The majority of beds used in private homes are equipped with slatted frames or also metal frames or can easily and at low cost be equipped with slatted frames. These mattress frames 3 usually lie on a supporting surface of the bedframe 2. The same principle of working may as well be applied to a bed where the mattress is directly lying on the bedframe 2.

Between the supporting surface of the bedframe 2 and the mattress frame 3, sensors 6 are located, preferably integrated, which carry the weight of the mattress frame 3, the mattress and the user and can be used for weight sensing and measuring of VBS like heart rate, breathing rate and activity. In order, not to enhance the height of the setup by introducing the sensors 6, the force acting on the sensors 6 is diverted to a space below the mattress frame 3, where in this space, the mattress frame 3 is unsupported by the bedframe 2, i.e. to a space, which is not located between the slatted frame 3 and bedframe 2. This is achieved by using a structure (see Figure 2) for direct mechanical coupling or by using an appropriate hinge mechanism.

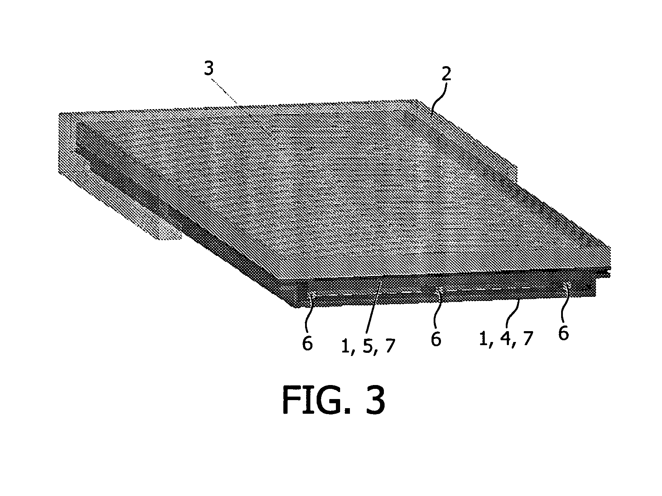

In Figures 2 and 3, a schematic side view and a schematic perspective view of an embodiment according to the invention are depicted. The structure 1 comprises a first sensor support 4 and a second sensor support 5, the sensors 6 being arranged between the first and second sensor support 4, 5. The first sensor support 4 is carried by the bedframe 2. The second sensor support 5 is carried via the sensors 6 by the first sensor support 4 and the mattress frame 3 is carried by the second sensor support 5. In this embodiment the first and second sensor support 4, 5 of structure 1 are formed as intermediate frames 7, spanning from one side of the bedframe 2 to the other. In Figure 2, a dimension 10 of the structure 1 between the bedframe 2 and the mattress frame 3 is depicted, which is substantially smaller than a distance 11 between the first sensor support 4 and the second sensor support 5. Thus the unutilized space below the mattress frame 3 is advantageously used for arranging the sensors 6.

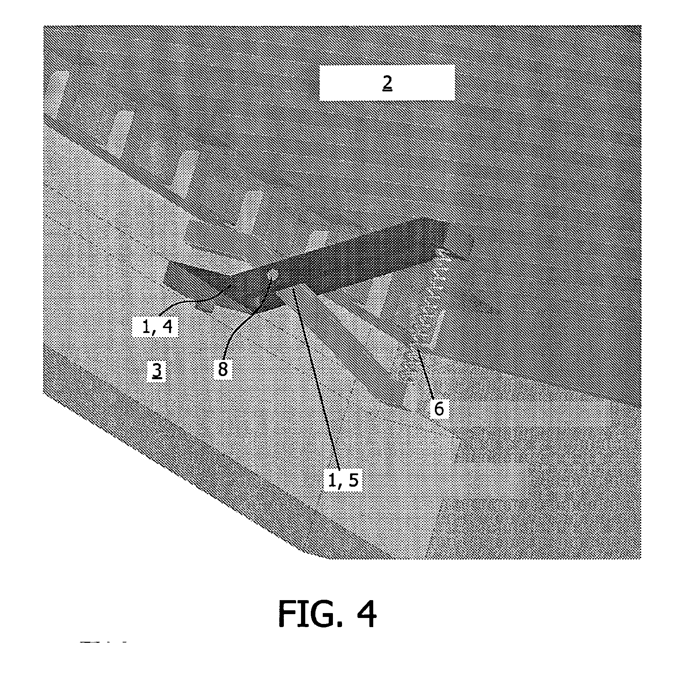

As can be seen from Figure 4, a perspective close-up perspective view of a sensor arrangement according to another embodiment is depicted, wherein instead of using an intermediate frame (Figures 2 and 3), the sensors 6 can be directly inserted in a similar scheme but as a single device. This embodiment of the sensor arrangement may be equipped in three, preferably four positions along the sides of the bedframe 2. The diversion of the force can be done directly using a structure 1 as for the intermediate frame (Figure 2) or using a hinge mechanism. Here, the first sensor support 4 and the second sensor support 5 are pivotally connected via pivot pin 8, thus forming a scissor-like mechanism.

The space in which the first sensor support 4 and the second sensor support 5 according to the invention extend, is enclosed by the mattress frame 3 from above, by vertical projections of the supporting surfaces of the bedframe 2 from the sides, and by the ground from below.

## Claims

1. Sensor arrangement for weight and/or movement sensing in a bed, comprising a structure (1) attachable between a bedframe (2) and a mattress frame (3), the bedframe (2) supporting the mattress frame (3) in an edging area, the structure comprising a first sensor support (4) and a second sensor support (5), the first sensor support and the second sensor support extending in a space below the mattress frame (3), where in the space, which is enclosed by the edging area and where the mattress frame is unsupported by the bedframe (2), at least one sensor (6) is arranged between the first sensor support (4) and the second sensor support (5) and
wherein the structure comprises load transmission means for transmitting forces exerted between the mattress frame (3) and the bedframe (2) to the first sensor support (4) and the second sensor support (5).

2. Sensor arrangement according to claim 1, wherein a dimension (10) of the structure (1) between the bedframe (2) and the mattress frame (3) is substantially smaller than a distance (11) between the first sensor support (4) and the second sensor support (5).

3. Sensor arrangement according to claim 1, wherein the first sensor support (4) is pivotally connected to the second sensor support (5).

4. Sensor arrangement according to claim 1, wherein the first sensor support (4) and/or the second sensor support (5) is formed as an intermediate frame (7).

5. Sensor arrangement according to claim 1, wherein a plurality of sensors (6) is arranged between the first sensor support (4) and the second sensor support (5).

6. Sensor arrangement according to claim 1, wherein the sensor (6) is a force detecting sensor.

7. Bed for sensing weight and/or movement of a user, comprising a bedframe (2) and a mattress frame (3), wherein at least one sensor arrangement according to claim 1 is attached between the bedframe and the mattress frame.

8. Bed according to claim 7, wherein the bed is retrofit with the sensor arrangement.

9. Method of sensing weight and/or movement of a user in a bed, comprising the steps of transferring forces exerted between a bedframe (2) and a mattress frame (3) of the bed to a space below the mattress frame, where in the space, which is enclosed by an edging area in which the bedframe (2) supports the mattress frame (3), the mattress frame is unsupported by the bedframe; and
measuring the forces by at least one sensor in said space below the mattress frame.

10. Use of a sensor arrangement according to claim 1 for sensing weight and/or movements of a user in a bed.

11. Use of a sensor arrangement according to claim 1 for equipping a bed comprising a bedframe (2) and a mattress frame (3) with a weight and/or movement sensing feature.

## Patentansprüche

1. Sensorgefüge für Gewichts- und/oder Bewegungswahrnehmung in einem Bett, umfassend eine Anordnung (1) befestigt zwischen einem Bettrahmen (2) und einem Matratzenboden (3), der Bettrahmen (2) tragend den Matratzenboden (3) in einem Randbereich, die Anordnung umfassend einen ersten Sensorträger (4) und einen zweiten Sensorträger (5), der erste Sensorträger und der zweite Sensorträger sich ausdehnend in einem Raum unter dem Matratzenboden (3), wobei der Raum, welcher umschlossen ist durch den Randbereich und wo der Matratzenboden (3) ungetragen ist durch den Bettrahmen (2), mindestens ein Sensor (6) ist platziert zwischen dem ersten Sensorträger (4) und dem zweitem Sensorträger (5) und
wobei die Anordnung Belastungsübertragungsmittel umfasst für Übertragung von Kräften ausgeübt zwischen dem Matratzenboden (3) und dem Bettrahmen (2) an den ersten Sensorträger (4) und den zweitem Sensorträger (5).

2. Sensorgefüge nach Anspruch 1, wobei eine Abmessung (10) der Anordnung (1) zwischen dem Bettrahmen (2) und dem Matratzenboden (3) wesentlich kleiner ist als eine Entfernung (11) zwischen dem ersten Sensorträger (4) und dem zweiten Sensorträger (5).

3. Sensorgefüge nach Anspruch 1, wobei der erste Sensorträger (4) drehpunktartig verbunden ist mit dem zweiten Sensorträger (5).

4. Sensorgefüge nach Anspruch 1, wobei der erste Sensorträger (4) und/oder der zweite Sensorträger (5) gestaltet ist als ein zwischenliegender Boden (7).

5. Sensorgefüge nach Anspruch 1, wobei eine Vielzahl an Sensoren (6) angeordnet ist zwischen dem ersten Sensorträger (4) und dem zweiten Sensorträger (5).

6. Sensorgefüge nach Anspruch 1, wobei der Sensor (6) ein krafterfassender Sensor ist.

7. Bett für Wahrnehmung des Gewichts und/oder der Bewegung eines Benutzers, umfassend einen Bettrahmen (3), wobei mindestens ein Sensorgefüge nach Anspruch 1 befestigt ist zwischen dem Bettrahmen und dem Matratzenboden.

8. Bett nach Anspruch 7, wobei das Bett nachgerüstet ist dem Sensorgefüge.

9. Verfahren der Wahrnehmung von Gewicht und/oder Bewegung eines Benutzers in einem Bett, umfassend die Schritte der Überleitung von Kräften ausgeübt zwischen einem Bettrahmen (2) und einem Matratzenboden (3) des Bettes auf einen Raum unter dem Matratzenboden, wo in dem Raum, welcher umschlossen ist durch einen Randbereich in welchem der Bettrahmen (2) den Matratzenboden (3) trägt, der Matratzenboden ungetragen ist durch den Bettrahmen; und
messend die Kräfte durch mindestens einen Sensor im benannten Raum unter dem Matratzenboden.

10. Gebrauch eines Sensorgefüges nach Anspruch 1 für das Wahrnehmen von Gewicht und/oder Bewegung eines Benutzers in einem Bett.

11. Gebrauch eines Sensorgefüges nach Anspruch 1 für das Ausstatten eines Bettes umfassend einen Bettrahmen (9) und einen Matratzenboden (3) mit einer Gewicht und/oder Bewegung wahrnehmenden Einrichtung.

## Revendications

1. Une disposition de capteurs pour mesurer le poids et/ou mouvement dans un lit, comprenant une structure (1) qui peut être attachée entre le cadre de lit (2) et le support du matelas (3), le cadre de lit (2) portant le support de matelas (3) sur une zone de bord, la structure comprenant un premier support de capteurs (4) et un second support de capteurs(5), le premier et le second support de capteurs s'étendant dans l'espace en dessous du support de matelas (3), cet espace, qui est enfermé par la zone de bord et où le support de matelas n'est pas porté par le cadre de lit (2), dans lequel au moins un capteur (6) est positionné entre le premier support de capteurs (4) et le second support de capteurs (5) et
dans laquelle la structure comprend un moyen de transmission de charge pour transmettre des forces générées entre le support de matelas (3) et le cadre de lit (2) vers le premier support de capteurs (4) et le second support de capteurs (5).

2. Disposition de capteurs selon revendication 1, dans laquelle une dimension (10) de la structure (1) entre le cadre de lit (2) et le support de matelas (3) est significativement plus petit qu'une distance (11) entre le premier support de capteurs (4) et le second support de capteurs (5).

3. Disposition de capteurs selon revendication 1, dans laquelle le premier support de capteurs (4) est raccordé de manière pivotante au second support de capteurs (5).

4. Disposition de capteurs selon revendication 1, dans laquelle le premier support de capteurs (4) et/ou le second support de capteurs (5) est sous la forme d'un cadre intermédiaire.

5. Disposition de capteurs selon revendication 1, dans laquelle une pluralité de capteurs (6) est positionné entre le premier support de capteurs (4) et le second support de capteurs (5).

6. Disposition de capteurs selon revendication 1, dans laquelle le capteur (6) est un capteur de forces.

7. Un lit pour mesurer le poids et/ou mouvement d'un utilisateur, comprenant un cadre de lit (2) et un support de matelas (3), dans laquelle au moins une disposition de capteurs selon revendication 1 est attachée entre le cadre de lit et le support de matelas.

8. Lit selon revendication 7, où le lit est transformé en ajoutant la disposition de capteurs.

9. Une méthode pour mesurer le poids et/ou mouvement d'un utilisateur dans un lit, comprenant les étapes de transfert de forces générées entre le cadre de lit (2) et le support de matelas (3) du lit vers un espace sous le support de matelas, où dans cet espace, qui est enfermé par une zone de bord dans laquelle de cadre de lit (2) porte le support de matelas (3), le support de matelas n'est pas porté par le cadre de lit; et
mesurant les forces par au moins un capteur dans ledit espace sous le support de matelas.

10. L'utilisation d'une disposition de capteur selon revendication 1 pour mesurer le poids et/ou mouvement d'un utilisateur dans un lit.

11. L'utilisation d'une disposition de capteur selon revendication 1 pour équiper un lit comprenant un cadre de lit (2) et un support de matelas (3) avec une fonction de mesure de poids et/ou mouvement.
